# EUROPEAN PATENT APPLICATION

(11) **EP 0 587 267 A1**
(43) Date of publication of application: **16.03.1994**
(21) Application number: 93303785.5
(22) Date of filing: 17.05.1993
(51) Int. Cl.: B26B 29/06, A61M 25/00

(54) **Guiding handtool for cutting a flexible tube**

(30) Priority: 23.05.1992 GB 9211064
(71) Applicant: The BOC Group plc, Windlesham Surrey GU20 6HJ (GB)
(72) Inventor: Bint, Stephen, 40 Godwin Road, Swindon, Wiltshire SN3 4XN (GB)
(74) Representative: Gough, Peter

(57) **Abstract**

A tool 1 for supporting plastics catheter 3 whilst it is being cut comprises a tubular support channel 2 in which the catheter 3 is received and a slit 4 for receiving a cutting blade. A handle 5 is attached to the support channel 2 as is a guard 6.

## Description

This invention relates to a tool for supporting a tubular object while it is cut. The tool is suitable for supporting catheters such as those formed from a plastic material, for example, polyurethane.

Catheters generally comprise a flexible tube through which fluids or gases may be admitted to or removed from the body of a patient. Catheter tubes are often introduced into the patient percutaneously. Catheter tubes are often cut to length so that the distance from the catheter tip to the fixation cuff may be customised to accommodate anatomical variations between patients.

Traditionally, a flexible catheter tube is cut using a pair of scissors or by placing the tube against a table or other surface and cutting through the catheter with a scalpel. However, these techniques of cutting the catheter tube nearly always result in an uneven cut and a catheter tip with a sharp point or ragged burr. Percutaneous introduction of a catheter with a sharp or burred catheter tip may cause damage to the body channels into which the catheter is being inserted. This damage may include surface abrasions and possibly punctures or rupture of the tissue of the body channel, for example, a vein. Furthermore, the sharp points or burrs may cause snagging as the catheter is introduced making the insertion procedure difficult to perform smoothly and efficiently.

In U.S. Patent 4195827 there is disclosed a tool for guiding the blade of a saw for cutting flexible conduit which includes a support channel for the conduit and a slit orientated across the support channel to receive and guide the saw blade.

Accordingly, the present invention provides a tool for supporting a tubular object while it is cut, which comprises a support channel in which the object can be received and a slit orientated across the support channel to receive a blade for cutting the object; characterised in that the support channel is closed along at least a portion of its length in the manner of a tube, a handle being attached to the support channel by which the tool may be gripped when in use and a guard attached to the support channel.

The tool of the invention allows the configuration of a cut to a tubular object, such as a catheter, to be controlled so that the formation of sharp points and burrs on the cut surfaces of the object are minimised. Problems associated with snagging of a catheter tip during percutaneous introduction are avoided and damage such as abrasions, cuts or puncture wounds to the internal surfaces of a body channel, for example, a vein, are substantially reduced or eliminated altogether.

The support channel is closed at least along a portion of its length in the manner of a tube. The tubular object is then inserted into the support channel and moved to a point where the object is required to be cut. The object is cut by using a blade, for example, a scalpel and cutting through the object which is exposed at the slit in the support channel. A tubular support channel which surrounds the object to be cut has the advantage of restricting excessive movement of the object.

Preferably, the slit is substantially perpendicular to the longitudinal axis of the support channel. It has been found that a cut which is substantially perpendicular and transverse to the support channel minimises the formation of burrs and sharp edges on the cut end of the object.

The guard may comprise two laterally extending portions between the slit in the support channel and the handle. Therefore, lacerations or cuts to the hand and fingers will be prevented by accidental slipping of the blade.

Preferably, the tool is made from a transparent material. Using material which is transparent has the advantage that it is possible to see exactly where the tubular object is within the tool.

The tool may be split through the handle and the support channel so that the transverse dimension of the support channel can be reduced by squeezing the handle. This is particularly advantageous when the support channel is tubular. A tubular object can be easily and freely inserted into the support channel to the desired point at which the tubular object is to be cut. The split or separated parts of the handle may then be squeezed which reduces the internal diameter or size of the support channel and thereby gripping the object. Movement of the object is prevented whilst it is cut. This also has the advantage that different sizes of tubular objects may be cut and gripped by the tool. It also has the advantage that objects with a range of sizes can be cut using the tool of the invention.

Conveniently, the tool includes a blade slidable from a first position in which the cutting edge of the blade is concealed to a second position in which the cutting edge of the blade is brought to bear upon and cut an object in the support channel. This allows accurate placement and cutting of the tubular object without letting go of the tubular object, for example, to pick up a scalpel to cut the object. The slidable blade may be fitted in the handle. Therefore, gripping and cutting may be facilitated in a simple movement by using only one hand.

The object, to be support by the tool, may be tubular. The tool is particularly suited to minimising sharp edges and burrs on the cut surfaces of flexible tubes used in catheters.

Preferably, the support channel is adapted to accept a tubular object less than about 10 mm, more preferably, less than about 4 mm.

Conveniently, the maximum dimension of the tool is less than about 50 mm. A tool of this size has the advantage that it may be incorporated into existing medical supply packs.

Embodiments of the present invention will now be described, by way of example only, with reference to the accompanying diagrammatic drawings, in which:
Figure 1 shows a perspective view of a tool for supporting a tubular object, such as a catheter, whilst it is cut;
Figure 2 shows an end view of another embodiment of tool with a split handle; and
Figure 3 shows a perspective view of a further embodiment of the tool.

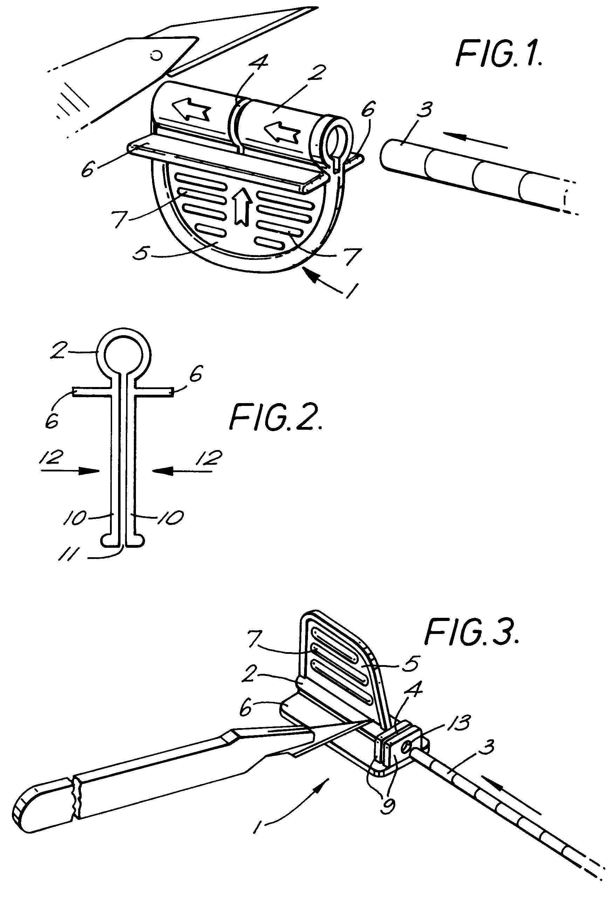

Referring to the drawings in detail, Figure 1 shows a preferred embodiment of a tool for supporting a tubular object, such as a catheter, whilst it is cut, generally indicated by reference numeral 1 and containing a tubular support channel 2 adapted to accept a flexible catheter tube 3, said support channel 2 having a slit 4 substantially perpendicular to the support channel axis, a handle 5 by which the device may be held and a finger guard 6. The finger guard 6 comprises two flat sections or wings protruding laterally from, and substantially perpendicular to, the handle 5 between the support channel 2 and the handle 5. Handle 5 may have ribs 7 formed on both sides of its surface to improve the grip obtained on the device.

To cut a tubular object such as a flexible catheter tube, the tube is inserted into the support channel 2 until the slit 4 is over the place or position at which the tube is to be cut. A blade, preferably a scalpel which is thin and sharp, is inserted into the slit 4 so that the cutting edge of the blade bears upon and cuts the tubular object within the support channel. The portion of the tube which is to be discarded is pushed out of the support channel and disposed of. The cut tube will have a cut end which is free from surface irregularities such as sharp points and burrs.

Figure 2 shows an end view of another embodiment of catheter cutting aid which comprises a support channel 2 adapted to accept a tubular object, split handles 10 with the split 11 extending through the supporting channel 2 and finger guards 6. This embodiment of catheter cutting aid has the advantage that a tubular object, such as a flexible catheter tube may be inserted into the supporting channel 2 and gripped in position. Gripping and locking of the object is achieved by pinching the handles 10 together in the direction of the arrows 12. The size of the slit 11 is reduced as is the diameter or size of the internal space of the support channel 2.

This in turn pinches the object or tube present in the support channel 2 preventing movement of the object because of the frictional contact created between the external surface of the object and the internal surface of the support channel. This prevents movement of the object. The object may be cut and then released by releasing the pressure applied to the handles 10.

Figure 3 shows a further embodiment of a tool for supporting a tubular object such as a catheter, whilst it is cut.

The tool 1 includes a support channel 2 closed along its length in the manner of a tube. A handle 5 formed with ribs 7 on both sides of its surface extends upwardly (as shown) from the support channel 2. A guard 6 comprises two flat sections or wings protruding laterally from the base of the support channel 2 and substantially perpendicular to the handle 5.

Adjacent the proximal end of the support tube 2 and mounted on the guard 6 are two guide plates 9 which between them define a slit 4 arranged substantially perpendicular to the longitudinal axis of the support channel 2. Each guide plate 5 has a hole 13 in alignment with the support channel 2.

In use, to cut a tubular object such as a flexible catheter tube 3, the handle 5 is grasped in one hand whilst the catheter tube 3 is inserted through the holes 13 in the guide plates 5 and into the support channel via its proximal end.

A blade, preferably a scalpel is inserted into the slit 4 so that the cutting edge of the blade bears down upon and cuts the catheter tube 3. The guard 6 limits downward (as shown) movement of the blade.

## Claims

1. A tool 1 for supporting an object 3 while it is cut comprising a support channel 2 in which the object 3 can be received and a slit 4 orientated across the support channel 2 to receive a blade for cutting the object 3 characterised in that the support channel 2 is closed along at least a portion of its length in the manner of a tube, a handle 5 being attached to the support channel 2 by which the tool 1 may be gripped when in use and a guard 6 attached to the support channel 2.

2. A tool as claimed in claim 1, characterised in that the slit 4 is substantially perpendicular to the longitudinal axis of the support channel 2.

3. A tool as claimed in claim 1 or 2, characterised in that the guard 6 comprises two laterally extending portions between the slit 4 in the support channel 2 and the handle 5.

4. A tool as claimed in any one of claims 1 to 3, characterised in that the tool 1 is made from a transparent material.

5. A tool as claimed in any one of claims 1 to 4, characterised in that the tool 1 is split through the handle 5 and the support channel 2 so that the transverse dimension of the support channel 2 can be reduced by squeezing the handle 5.

6. A tool as claimed in any one of claims 1 to 5, characterised in that the tool 1 includes a blade slidable from a first position in which the cutting edge of the blade is concealed to a second position in which the cutting edge of the blade is brought to bear upon and cut an object 3 in the support channel 2.

7. A tool as claimed in any one of claims 1 to 6, characterised in that the object 3, to be supported by the tool 1, is tubular.

8. A tool as claimed in any one of claims 1 to 7, characterised in that the support channel 2 is adapted to accept a tubular object 3 less than about 10 mm, more preferably, less than about 4 mm.
